Europäisches Patentamt

European Patent Office

Office européen des brevets

(19)

(11) Numéro de publication: **0 305 276**
**A2**

(12) **DEMANDE DE BREVET EUROPEEN**

(21) Numéro de dépôt: **88402120.5**

(22) Date de dépôt: **18.08.88**

(51) Int. Cl.⁴: **A 61 K 31/425**

(30) Priorité: **25.08.87 FR 8711882**

(43) Date de publication de la demande:
**01.03.89 Bulletin 89/09**

(84) Etats contractants désignés:
**AT BE CH DE ES FR GB GR IT LI LU NL SE**

(71) Demandeur: **RHONE-POULENC SANTE**
**20, avenue Raymond Aron**
**F-92160 Antony (FR)**

(72) Inventeur: **Gueremy, Claude**
**3 Rue Daumesnil**
**F-78800 Houilles (FR)**

**Maillard, Françoise**
**63 Elysée 2**
**F-78170 La Celle Saint Cloud (FR)**

**Musch, Bruno**
**53 Avenue Foch**
**F-75016 Paris (FR)**

(74) Mandataire: **Savina, Jacques et al**
**RHONE-POULENC INTERSERVICES Service Brevets**
**Pharma 25, quai Paul Doumer**
**F-92408 Courbevoie Cédex (FR)**

(54) Application de l'amino-2 trifluorométhoxy-6 benzothiazole pour obtenir un médicament destiné au traitement de la schizophrénie.

(57) Application de l'amino-2 trifluorométhoxy-6 benzothiazole pour obtenir un médicament destiné au traitement de la schizophrénie et plus particulièrement ses formes déficitaires.

EP 0 305 276 A2

## Description

# APPLICATION DE L'AMINO-2 TRIFLUOROMETHOXY-6 BENZOTHIAZOLE POUR OBTENIR UN MEDICAMENT DESTINE AU TRAITEMENT DE LA SCHIZOPHRENIE

La présente invention concerne l'application de l'amino-2 trifluorométhoxy-6 benzothiazole ou un sel pharmaceutiquement acceptable de ce composé pour obtenir un médicament destiné au traitement de la schizophrénie et plus particulièrement ses formes déficitaires.

Du brevet européen 50 551, il est connu que l'amino-2 trifluorométhoxy-6 benzothiazole ou un sel pharmaceutiquement acceptable de ce composé est utile comme médicament anticonvulsivant, anxiolytique et hypnotique.

Il a maintenant été trouvé que l'amino-2 trifluorométhoxy-6 benzothiazole ou un sel pharmaceutiquement acceptable de ce composé est utile dans le traitement de la schizophrénie et plus particulièrement les formes déficitaires de la schizophrénie.

Cette nouvelle application thérapeutique a été déterminée chez l'homme en monothérapie stricte. 26 patients schizophrènes ont été traités à la dose de 150 mg par jour par voie orale pendant 28 jours.

L'efficacité de ce composé a été déterminée selon les échelles d'évaluation suivantes :
- SANS (échelle d'évaluation des symptômes déficitaires d'ANDREASEN), N.C. ANDREASEN, Arch. Gen. Psychiatry vol 39, 784-788 (1982) ;
- BPRS (Brief Psychiatric Rating Scale), P. PICHOT et al, Revue de Psychologie Appliquée vol 19, N°3, 217-232 (1969) ;
- Hamilton Anxiety Scale, P. BECH et al, Acta Psychiatrica Scandinavica supplementum N° 326, vol 73, p.9, 19-22 1986 ;
- CGI (Clinical Global Impression), ECDEU assessment manual, US department of Health, Education and Welfare, p.218-221.

Les principaux symptômes améliorés sont :
- reprise de contact avec l'entourage,
- malade plus vif tant du point de vue kinétique que psychique,
- resocialisation,
- amélioration du sommeil.

Aucun effet secondaire clinique n'a été observé.

L'amino-2 trifluorométhoxy-6 benzothiazole peut être préparé selon le procédé décrit dans le brevet EP 50 551.

Les médicaments utiles dans le traitement de la schizophrénie sont constitués par l'amino-2 trifluorométhoxy-6 benzothiazole ou un sel pharmaceutiquement acceptable de ce composé à l'état pur ou sous forme d'une composition dans laquelle il est associé à tout autre produit pharmaceutiquement compatible, pouvant être inerte ou physiologiquement actif. Ces médicaments peuvent être utilisés par voie orale, parentérale ou rectale.

Comme compositions solides pour administration orale peuvent être utilisés des comprimés, pilules, poudres (capsules de gélatine, cachets) ou granulés. Dans ces compositions, le principe actif est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les doses dépendent de l'effet recherché, de la durée du traitement et de la voie d'administration utilisée ; elles sont généralement comprises entre 50 et 500 mg par jour par voie orale pour un adulte avec des doses unitaires allant de 10 à 50 mg de substance active.

D'une façon générale, le médecin déterminera la posologie appropriée en fonction de l'âge, du poids et de tous les autres facteurs propres au sujet à traiter.

Les exemples suivants illustrent des compositions pharmaceutiques utilisables pour le traitement de la schizophrénie.

### Exemple A

On prépare, selon la technique habituelle, des gélules dosées à 50 mg de produit actif ayant la composition suivante :
- amino-2 trifluorométhoxy-6 benzothiazole    50 mg

- cellulose    18 mg
- lactose    55 mg
- silice colloïdale    1 mg
- carboxyméthylamidon sodique    10 mg
- talc    10 mg
- stéarate de magnésium    1 mg

## Exemple B

On prépare, selon la technique habituelle, des comprimés dosés à 50 mg de produit actif ayant la composition suivante :
- amino-2 trifluorométhoxy-6 benzothiazole    50 mg
- lactose    104 mg
- cellulose    40 mg
- polyvidone    10 mg
- carboxyméthylamidon sodique    22 mg
- talc    10 mg
- stéarate de magnésium    2 mg
- silice colloïdale    2 mg
- mélange d'hydroxyméthylcellulose, glycérine, oxyde de titane (72-3,5-24,5)    q.s.p. 1 comprimé pelliculé terminé à 245 mg

## Exemple C

On prépare une solution injectable contenant 10 mg de produit actif ayant la composition suivante :
- amino-2 trifluorométhoxy-6 benzothiazole    10 mg
- acide benzoïque    80 mg
- alcool benzylique    0,06 cm³
- benzoate de sodium    80 mg
- éthanol à 95 %    0,4 cm³
- hydroxyde de sodium    24 mg
- propylèneglycol    1,6 cm³
- eau    q.s.p. 4 cm³

**Revendications**

1 - Application de l'amino-2 trifluorométhoxy-6 benzothiazole ou un sel pharmaceutiquement acceptable de ce composé pour obtenir un médicament destiné au traitement de la schizophrénie et plus particulièrement ses formes déficitaires.